# EUROPEAN PATENT SPECIFICATION

(11) **EP 2 467 124 B1**
(45) Date of publication and mention of the grant of the patent: **17.08.2016**
(21) Application number: 10739805.9
(22) Date of filing: 30.07.2010
(51) Int. Cl.: A61K 8/85, A61Q 5/12

(54) **HAIR CARE COMPOSITIONS COMPRISING FIRST AND SECOND SUCROSE POLYESTERS**
HAARBEHANDLUNGSMITTEL ENTHALTEND ZWEI SUCROSEPOLYESTER
COMPOSITIONS DE SOINS CAPILLAIRES COMPRENANT UN PREMIER ET UN SECOND POLYESTER DE SUCROSE

(30) Priority: 20.08.2009 US 235439 P
(43) Date of publication of application: 27.06.2012
(73) Proprietor: The Procter & Gamble Company, Cincinnati, OH 45202 (US)
(72) Inventor: SUNKEL, Jorge, Max, Cincinnati Ohio 45241 (US); NAEMURA, Kenji, Tatsuno Hyogo 671-1602 (JP)
(74) Representative: Briatore, Andrea
(86) International application number: PCT/US2010/043803
(87) International publication number: WO 2011/022187

(56) References cited:
- WO-A2-2009/107062
- US-A1- 2002 071 819

## Description

### FIELD OF THE INVENTION

The present invention relates to hair care compositions for delivering a conditioning benefit, comprising two different sucrose polyesters.

### BACKGROUND OF THE INVENTION

Human hair becomes dry and/or damaged due to the surrounding environment, styling, drying, and/or coloring or otherwise chemically treating the hair.

A variety of approaches have been developed to condition the hair. A common method of providing conditioning benefit is through the use of hair care compositions containing conditioning agents such as cationic surfactants and polymers, high melting point fatty compounds, low melting point oils, silicone compounds, and mixtures thereof. Silicones are often used as a conditioning active for a number of hair care compositions, especially for providing conditioning benefits such as friction reduction and smoothness on dry hair. However, there exists a trend of the rising costs of such silicone compounds. There also exists a trend of using materials derived from natural resource rather than those from petroleum such as silicone materials, from environmental standpoints and/or consumer acceptance standpoints.

Based on the foregoing, there remains a need for a conditioning active which can provide conditioning benefits to hair, especially friction reduction and/or smoothness on dry hair, which can replace, or be used in combination with silicone, or other conditioning active.

Alternatively, there remains a need for hair care composition which can provide hair manageability benefit, such as volume control, easy of combing, and/or hair alignment, etc.

None of the existing art provides all of the advantages and benefits of the present invention.

### SUMMARY OF THE INVENTION

The present invention relates to a hair care composition comprising:
a) at least one first sucrose polyester has a melting point greater than 50°C and an iodine value (IV) of from 0.1 to 10
b) at least one second sucrose polyester has a melting point lower than 10°C and an iodine value (IV) of from 100 to 150°C and
c) an aqueous carrier.

The hair care composition of the present invention provides conditioning benefits, especially friction reduction/smoothness on dry hair and/or hair manageability.

The present invention also relates to use of a first sucrose polyester, which has a melting point of greater than 50°C and an iodine value (IV) of from 0.1 to 10 together with a second sucrose polyester, which has a melting point lower than 10°C and an iodine value (IV) of from 100 to 150, to provide a friction reduction between hair. These and other features, aspects, and advantages of the present invention will become evident to those skilled in the art from a reading of the present disclosure.

### DETAILED DESCRIPTION OF THE INVENTION

While the specification concludes with claims which particularly point out and distinctly claim the invention, it is believed the present invention will be better understood from the following description.

All percentages, parts and ratios are based upon the total weight of the compositions of the present invention, unless otherwise specified. All such weights as they pertain to listed ingredients are based on the active level and, therefore, do not include solvents or by-products that may be included in commercially available materials, unless otherwise specified. The term "weight percent" may be denoted as "wt. %" herein.

All molecular weights as used herein are weight average molecular weights expressed as grams/mole, unless otherwise specified.

Herein, "comprising" means that other steps and other ingredients which do not affect the end result can be added. This term encompasses the terms "consisting of" and "consisting essentially of". The compositions and methods/processes of the present invention can comprise, consist of, and consist essentially of the elements and limitations of the invention described herein, as well as any of the additional or optional ingredients, components, steps, or limitations described herein.

Herein, "mixtures" is meant to include a simple combination of materials and any compounds that may result from their combination.

The term "hair care composition" as used herein shall include shampoos, rinse out conditioners, leave in conditioners, styling products, and/or hair colorants.

The term "polymer" as used herein shall include materials whether made by polymerization of one type of monomer or made by two (i.e., copolymers) or more types of monomers.

The term "suitable for application to human hair" as used herein means that the compositions or components thereof so described are suitable for use in contact with human hair and the scalp and skin without undue toxicity, incompatibility, instability, allergic response, and the like.

### SUCROSE POLYESTERS

The hair care compositions of the present invention comprise at least one first sucrose polyester and at least one second sucrose polyester. Sucrose polyesters are derived from a natural resource and therefore, the use of sucrose polyesters as the conditioning active can result in a positive environmental impact. It is believed that the use of the first and second sucrose polyesters provide improved conditioning benefits especially friction reduction/smoothness on dry hair and/or hair manageability, compared to the use of each of the first or second sucrose polyester.

It is also believed that, the above improved conditioning benefit may be due to improved deposition of the sucrose polyesters when using both of the first and second sucrose polyesters, compared to deposition when using each of the first or second sucrose polyester.

Sucrose polyesters are polyester materials, having multiple substitution positions (up to 8 positions) around the sucrose backbone coupled with the chain length, saturation, and derivation variables of the fatty chains.

### Melting point

The first sucrose polyester useful herein has a melting point greater than 50°C. The first sucrose polyester is preferably in a solid form at a room temperature.

The second sucrose polyester useful herein has a melting point lower than 10°C, preferably lower than 0°C. The second sucrose polyester is preferably in a liquid form at a room temperature.

### Iodine Value (IV)

The first sucrose polyester useful herein has a saturation degree which can be expressed by an iodine value ("IV") of from 0.1 to 10. The first sucrose polyester is preferably highly saturated.

The second sucrose polyester useful herein has a saturation degree which can be expressed by an iodine value ("IV") of from 100 to 150. The second sucrose polyester is preferably highly unsaturated.

### Esterification Degree

Generally, sucroses have 8 positions around the sucrose backbone which can be esterified. Thus, sucrose polyesters useful herein are those having multiple esterified positions, i.e., from about 4 to 8, preferably from 6 to 8, more preferably 8, in view of chemical stability against hydrolysis of the ester linkages. Higher esterification degrees may also be preferred in view of providing dry conditioning benefits due to more hydrophobicity thereof. Esterification Degree used herein means that the number of esterified positions.

### Fatty chain length

The first and second sucrose polyesters useful herein respectively have a fatty chain length of from C12 to C30, preferably from C14 to C26, more preferably from C16 to C22, for providing dry conditioning benefits. In the present invention, "sucrose polyesters having a fatty chain length" means that: at least 50%, preferably at least 60%, more preferably at least 70%, still more preferably at least 80% of the fatty chains of the sucrose polyesters have the fatty chain length which is specified above.

### Commercial examples

Commercial examples of such first sucrose polyesters useful herein include, for example: that available as Sefose 1618H from P&G Chemicals, having a melting point of 60°C, IV of 3, Esterification Degree of 8, an alkyl distribution containing 87% of C18 and 13% of C16; and that available as Sefose 2275C from P&G Chemicals, having a melting point of 70°C, IV of 5, Esterification Degree of 8, and an alkyl distribution containing 80% of C22 and 20% of C18. Commercial examples of such second sucrose polyesters useful herein include, for example: that available as Sefose 1618U from P&G Chemicals, having a melting point of -5°C, IV of 135, Esterification Degree of 8, and an alkyl distribution containing 86% of C18 and 12% of C16 and 2% of others; and that available as Sefose 1618S from P&G Chemicals, having a melting point of 10°C, IV of 85, Esterification Degree of 6, and an alkyl distribution containing 87% of C18 and 13% of C16.

### Level in the composition

Total amount of the first and second sucrose polyesters in the composition is preferably from 0.1% to 10%, more preferably from 0.3% to 5%, still more preferably from 0.5% to 2.5%, even more preferably from 0.6% to 2.0% by weight of the composition, in view of providing balanced conditioning benefits and hair manageability benefits.

### Weight ratio

The weight ratio of the first sucrose polyester to the second sucrose polyester is from 1:1.6 to 1:3. Especially in view of providing hair manageability, the weight ratio of the first sucrose polyester to the second sucrose polyester is preferably from about 1:0.7 to about 1:1.5.

### SUCROSE POLYESTER PREMIX/BLEND

The first and second sucrose polyesters can be added independently and separately to the carrier, concurrently and separately to the carrier, or concurrently as a homogeneous blend to the carrier. When forming a homogeneous blend, the blend can have the following melting points and rheology profiles. Other features such as a total amount in the composition and weight ratios are same as above described.

### Melting point as premix/blend

In the present invention, in view of providing friction reduction benefit, it is preferred that the blend has a melting point of from 30°C to 65°C, more preferably from 35°C to 60°C, still more preferably from 37°C to 58°C. Alternatively, in view of providing hair manageability, it is preferred that the blend has a melting point of from 40°C to 72°C, more preferably from 45°C to 65°C, still more preferably from 47°C to 60°C.

### Rheology as premix/blend

In the present invention, it is preferred that the blend has a certain rheological profile, especially in view of providing hair manageability benefits. Such rheological profiles are as follows:
G' value at 0.01Hz of from 100Pa to 50,000Pa, more preferably from 1,000Pa to 40,000Pa, still more preferably from 5,000Pa to 30,000Pa;
G" value at 0.01Hz of from 100Pa to 100,000Pa, more preferably from 1,000Pa to 50,000Pa, still more preferably from 5,000Pa to 40,000Pa;
G' value at 1Hz of from 1,000Pa to 1,000,000Pa, more preferably from 5,000Pa to 900,000Pa, still more preferably from 10,000Pa to 800,000Pa; and
G" value at 1Hz of from 10,000Pa to 3,000,000Pa, more preferably from 50,000Pa to 1,000,000Pa, still more preferably from 100,000Pa to 800,000Pa.

G' and G" are measured by the following method:
The blends are prepared by placing the amounts and types of sucrose polyesters, at the specified ratio, in a stainless steel beaker which is heated in a water bath at 75°C or at a temperature any solid sucrose polyester present in the blend melts and is intimately mixed with all other sucrose polyesters to create a homogenous mixture. After this, the beaker is removed from the bath and allowed to cool at room temperature. Samples from these blends are transferred to the rheometer for G' and G" measurements. Each blend is carefully transferred to a rheometer plate while avoiding applying vigorous shear to each blend composition. A suitable rheometer for use in the present method is the Haake RS-150 RheoStress Rheometer interfaced with a computer having suitable software that provides data recordation and analysis. The test is performed at temperature of 35°C and the equilibration time is 3 minute. The rheometer is configured with a 35 mm diameter, 4 degree steel cone at a gap setting to the plate of 140 µm as measured from the center of the cone. The angular frequency is applied, starting at 0.01 Hz, where G' and G" are measured three times and the average of G' and G" are recorded by the software. Similarly, these measurements are repeated at each decade after 0.01 Hz and up to at least 1Hz. All measurements were performed at a constant stress of 1 Pa.

### Highly preferred blend

Preferred blends in view of providing friction reduction benefits include, for example, 1:2.3 blend of Sefose 1618H and Sefose 1618U, the blend having a melting point of 54°C.

Preferred blends in view of providing hair manageability includes, for example, 1:1 blend of Sefose 1618H and Sefose 1618U, the blend having a melting point of 50°C, and the blend having G' and G" values at 0.01Hz of 10,000Pa and 24,000Pa and G' and G" values at 1Hz of 56,000Pa and 395,000Pa.

### METHOD OF PREPARATION

The first and second sucrose polyesters can be added independently and separately to the carrier, concurrently and separately to the carrier, or concurrently as a homogeneous blend to the carrier. The first and/or second sucrose polyesters, independently or as a blend, can be emulsified before adding them to the carrier.

In any case, it is preferred to add sucrose polyesters to the carrier at a temperature which is higher than melting points thereof. For example, it's preferred to add the first sucrose polyester to the carrier at a temperature which is higher than the melting point of the first sucrose polyester, and it's preferred to add the second sucrose polyester to the carrier at a temperature which is higher than the melting point of the second sucrose polyester. When the first and second sucrose polyesters are premixed to form a homogenous blend, it is preferred to add the blend to the carrier at a temperature which is higher than the melting point of the blend.

When premixing, it's also preferred to heat them up until they become a homogeneous mixture, for example, to a temperature which is above the melting point of the first sucrose polyesters.

When premixing, and when composition further comprises a fatty alcohol having from 14 to 30 carbon atoms and a cationic surfactant which form a gel matrix together with the aqueous carrier, it is further preferred that the blend is added prior to the formation of gel matrix.

By the above preferred method of preparation, it is believed that the compositions provide better dry conditioning benefits and homogeneous product appearance.

### CATIONIC SURFACTANT

The composition of the present invention preferably contains a cationic surfactant. The cationic surfactant can be included in the composition at a level by weight of from 0.2% to 10%, preferably from 0.3% to 8%, more preferably from 0.4% to 5%, in view of providing conditioning benefits.

A variety of cationic surfactants can be used herein. Such cationic surfactants include, for example: mono-long alkyl cationic surfactants having one long alkyl chain, for example, that having from 12 to 40 carbon atoms, such as mono-long alkyl quaternary ammonium salts and mono-long alkyl amines, mono-long alkyl amidoamines and salts thereof; and di-long alkyl cationic surfactants having two long alkyl chains, for example those having from 12 to 40 carbon atoms, such as dicetyl dimethyl ammonium chloride and distearyl dimethyl ammonium chloride.

Among a variety of the cationic surfactant, a cationic surfactant system comprising a mono-long alkyl cationic surfactant and a di-long alkyl cationic surfactant may be preferred. In the system, it is preferred that the weight ratio of the di-long alkyl cationic surfactant to the mono-long alkyl cationic surfactant is within the range of from 1:1 to 1:20, more preferably from 1:1 to 1:15, still more preferably from 1:1 to 1:10, in view of providing conditioning benefits.

Among a variety of the cationic surfactant systems, the following systems may be preferred: the system comprising mono-long alkyl cationic surfactants selected from the group consisting of behenyl trimethyl ammonium methyl sulfate, behenyl trimethyl ammonium ethyl sulfate, and mixtures thereof, and di-long alkyl cationic surfactants selected from the group consisting of dicetyl dimethyl ammonium chloride, distearyl dimethyl ammonium chloride, and mixtures thereof.

The compounds, which can be used in the cationic surfactant system of the present invention, are explained below in detail.

### Mono-long alkyl quaternized ammonium salt cationic surfactant

One of the preferred cationic surfactants of the present invention is a salt of a mono-long alkyl quaternized ammonium and an anion, wherein the anion is selected from the group consisting of halides such as chloride and bromide, C1-C4 alkyl sulfate such as methosulfate and ethosulfate, and mixtures thereof.

The mono-long alkyl quaternized ammonium salts useful herein are those having the formula (I): 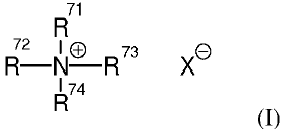 wherein one of R⁷¹, R⁷² , R⁷³ and R⁷⁴ is selected from an aliphatic group of from 16 to 40 carbon atoms or an aromatic, alkoxy, polyoxyalkylene, alkylamido, hydroxyalkyl, aryl or alkylaryl group having up to 40 carbon atoms; the remainder of R⁷¹, R⁷² , R⁷³ and R⁷⁴ are independently selected from an aliphatic group of from 1 to 8 carbon atoms or an aromatic, alkoxy, polyoxyalkylene, alkylamido, hydroxyalkyl, aryl or alkylaryl group having up to 8 carbon atoms; and X- is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, *e.g.,* those of 16 carbons, or higher, can be saturated or unsaturated. Preferably, one of R⁷¹, R⁷², R⁷³ and R⁷⁴ is selected from an alkyl group of from 16 to 40 carbon atoms, more preferably from 18 to 26 carbon atoms, still more preferably from 22 carbon atoms; and the remainder of R⁷¹, R⁷², R⁷³ and R⁷⁴ are independently selected from CH₃, C₂H₅, C₂H₄OH, CH₂C₆H₅, and mixtures thereof. It is believed that mono-long alkyl quaternized ammonium salts can provide improved hydrophobicity and smooth feel on dry hair, compared to amine or amine salt cationic surfactants.

Among them, more preferred cationic surfactants are those having a longer alkyl group, i.e., C18-22 alkyl group. Such cationic surfactants include, for example, behenyl trimethyl ammonium chloride, methyl sulfate or ethyl sulfate, and stearyl trimethyl ammonium chloride, methyl sulfate or ethyl sulfate. More preferred are behenyl trimethyl ammonium methyl sulfate or ethyl sulfate and stearyl trimethyl ammonium methyl sulfate or ethyl sulfate, and still more preferred is behenyl trimethyl ammonium methyl sulfate or ethyl sulfate. It is believed that; cationic surfactants having a longer alkyl group provide improved smoothness and soft feeling on wet and dry hair, compared to cationic surfactant having a shorter alkyl group. It is also believed that such cationic surfactants can provide reduced irritation, compared to cationic surfactants having a shorter alkyl group.

### Mono-long alkyl amine cationic surfactant

Mono-long alkyl amines are also suitable as cationic surfactants. Primary, secondary, and tertiary fatty amines are useful. Particularly useful are tertiary amido amines having an alkyl group of from 12 to 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al. These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The amines herein are preferably partially neutralized with any of the acids at a molar ratio of the amine to the acid of from 1 : 0.3 to 1 : 2, more preferably from 1 : 0.4 to 1 : 1.

### Di-long alkyl quaternized ammonium salt

Di-long alkyl quaternized ammonium salts useful herein are those having the formula (II): 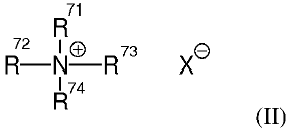 wherein two of R⁷¹, R⁷², R⁷³ and R⁷⁴ is selected from an aliphatic group of from 16 to 40 carbon atoms or an aromatic, alkoxy, polyoxyalkylene, alkylamido, hydroxyalkyl, aryl or alkylaryl group having up to 40 carbon atoms; the remainder of R⁷¹, R⁷², R⁷³ and R⁷⁴ are independently selected from an aliphatic group of from 1 to 8 carbon atoms or an aromatic, alkoxy, polyoxyalkylene, alkylamido, hydroxyalkyl, aryl or alkylaryl group having up to about 8 carbon atoms; and X- is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, *e.g.,* those of about 16 carbons, or higher, can be saturated or unsaturated. Preferably, one of R⁷¹, R⁷² R⁷³ and R⁷⁴ is selected from an alkyl group of from 16 to 40 carbon atoms, more preferably from 16 to 26 carbon atoms; and the remainder of R⁷¹, R⁷² R⁷³ and R⁷⁴ are independently selected from CH₃, C₂H₅, C₂H₄OH, CH₂C₆H₅, and mixtures thereof. Preferably, the anion is selected from the group consisting of halides such as chloride and mixtures thereof.

Such di-long alkyl quaternized ammonium salts useful herein include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

### HIGH MELTING POINT FATTY COMPOUND

The composition of the present invention preferably contains a high melting point fatty compound. The high melting point fatty compound can be included in the composition at a level of from 1% to 15%, preferably from 3% to 10%, more preferably from 5% to 8% by weight of the composition, in view of providing conditioning benefits such as slippery feel during the application to wet hair, softness and moisturized feel on dry hair.

The high melting point fatty compound useful herein have a melting point of 25°C or higher, and is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are preferably used in the composition of the present invention. The fatty alcohols useful herein are those having from 14 to 30 carbon atoms, preferably from 16 to 22 carbon atoms. These fatty alcohols are saturated and can be straight or branched chain alcohols. Preferred fatty alcohols include, for example, cetyl alcohol, stearyl alcohol, behenyl alcohol, and mixtures thereof.

High melting point fatty compounds of a single compound of high purity are preferred. Single compounds of pure fatty alcohols selected from the group of pure cetyl alcohol, stearyl alcohol, and behenyl alcohol are highly preferred. By "pure" herein, what is meant is that the compound has a purity of at least 90%, preferably at least 95%. These single compounds of high purity provide good rinsability from the hair when the consumer rinses off the composition.

### AQUEOUS CARRIER

The conditioning composition of the present invention comprises an aqueous carrier. The level and species of the carrier are selected according to the compatibility with other components, and other desired characteristic of the product. Generally, the compositions of the present invention comprise from 20% to 99%, preferably from 30% to 95%, and more preferably from 80% to 95% water.

The carrier useful in the present invention includes water and water solutions of lower alkyl alcohols and polyhydric alcohols. The lower alkyl alcohols useful herein are monohydric alcohols having 1 to 6 carbons, more preferably ethanol and isopropanol. The polyhydric alcohols useful herein include propylene glycol, hexylene glycol, glycerin, and propane diol.

Preferably, the aqueous carrier is substantially water. Deionized water is preferably used. Water from natural sources including mineral cations can also be used, depending on the desired characteristic of the product.

### GEL MATRIX

Preferably, the above cationic surfactants, together with high melting point fatty compounds and an aqueous carrier, form a gel matrix in the composition of the present invention.

The gel matrix is suitable for providing various conditioning benefits such as slippery feel during the application to wet hair and softness and moisturized feel on dry hair. In view of providing the above gel matrix, the cationic surfactant and the high melting point fatty compound are contained at a level such that the weight ratio of the cationic surfactant to the high melting point fatty compound is in the range of, preferably from 1:1 to 1:10, more preferably from 1:1 to 1:6, more preferably form 1:1 to 1:4.

Preferably, especially when the composition contains cationic surfactant and/or gel matrix, the composition of the present invention is substantially free of anionic surfactants and anionic polymers, in view of compatibility with cationic surfactants, and stability of the gel matrix when formed by cationic surfactants and high melting point fatty compounds. In the present invention, "the composition being substantially free of anionic surfactants and anionic polymers" means that: the composition is free of anionic surfactants and anionic polymers; or, if the composition contains anionic surfactants and anionic polymers, the level of such anionic surfactants and anionic polymers is very low. In the present invention, a total level of such anionic surfactants and anionic polymers, if included, preferably 1% or less, more preferably 0.5% or less, still more preferably 0.1% or less by weight of the composition. Most preferably, the total level of such anionic surfactants and anionic polymers is 0% by weight of the composition.

### SILICONE COMPOUND

The compositions of the present invention may contain a silicone compound. The silicone compounds herein can be used at levels by weight of the composition of preferably from 0.1% to 20%, more preferably from 0.15% to 10%, still more preferably from 0.2% to 8%.

The silicone compounds useful herein, as a single compound, as a blend or mixture of at least two silicone compounds, or as a blend or mixture of at least one silicone compound and at least one solvent, have a viscosity of preferably from 1,000 to 2,000,000mPa·s at 25°C.

The viscosity can be measured by means of a glass capillary viscometer as set forth in Dow Corning Corporate Test Method CTM0004, July 20, 1970. Suitable silicone fluids include polyalkyl siloxanes, polyaryl siloxanes, polyalkylaryl siloxanes, polyether siloxane copolymers, amino substituted silicones, quaternized silicones, and mixtures thereof. Other nonvolatile silicone compounds having conditioning properties can also be used.

Preferably, the silicone compounds have an average particle size of from 1 microns to 50 microns, in the composition.

Preferably, silicone compounds useful herein include amino substituted materials. Preferred aminosilicones include, for example, those which conform to the general formula (III):

(R₁)ₐG₃₋ₐ-Si-(-OSiG₂)ₙ-(-OSiG_{b}(R₁)_{2-b})m-O-SiG₃₋ₐ(R₁)ₐ

wherein G is hydrogen, phenyl, hydroxy, or C₁-C₈ alkyl, preferably methyl; a is an integer having a value from 1 to 3, preferably 1; b is 0, 1 or 2, preferably 1; n is a number from 1 to 2,000, preferably from 100 to 2,000, more preferably from 300 to 1,800; m is an integer from 0 to 1,999, preferably from 0 to 10, more preferably 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A⁻; -N(R₂)CH₂-CH₂-NR₂H₂A⁻; wherein R₂ is hydrogen, phenyl, benzyl, or a saturated hydrocarbon radical, preferably an alkyl radical from C₁ to C₂₀; A is a halide ion.

One highly preferred amino silicones are those corresponding to formula (III) wherein m=0, a=1, q=3, G=methyl, n is preferably from 1500 to 1700, more preferably 1600; and L is -N(CH₃)₂ or -NH₂, more preferably -NH₂. Another highly preferred amino silicones are those corresponding to formula (III) wherein m=0, a=1, q=3, G=methyl, n is preferably from 400 to 600, more preferably 500; and L is -N(CH₃)₂ or -NH₂, more preferably -NH₂. Such highly preferred amino silicones can be called as terminal aminosilicones, as one or both ends of the silicone chain are terminated by nitrogen containing group. It is believed that, such terminal aminosilicone can provide balanced benefit between conditioning benefits and clean feel, compared to other silicones such as graft aminosilicones and silicones having no amino substitution.

The above aminosilicones, when incorporated into the composition, can be mixed with solvent having a lower viscosity. Such solvents include, for example, polar or non-polar, volatile or non-volatile oils. Such oils include, for example, silicone oils, hydrocarbons, and esters. Among such a variety of solvents, preferred are those selected from the group consisting of non-polar, volatile hydrocarbons, volatile cyclic silicones, non-volatile linear silicones, and mixtures thereof. The non-volatile linear silicones useful herein are those having a viscosity of from about 1 to 20,000 centistokes, preferably from 20 to 10,000 centistokes at 25°C. Among the preferred solvents, highly preferred are non-polar, volatile hydrocarbons, especially non-polar, volatile isoparaffins, in view of reducing the viscosity of the aminosilicones and providing improved hair conditioning benefits such as reduced friction on dry hair. Such mixtures have a viscosity of preferably from 1,000mPa·s to 100,000mPa·s, more preferably from 5,000mPa·s to 50,000mPa·s.

The other silicone compounds useful herein include polyalkyl or polyaryl siloxanes with the following structure: 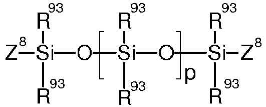 wherein R⁹³ is alkyl or aryl, and p is an integer from 7 to 8,000. Z⁸ represents groups which block the ends of the silicone chains. The alkyl or aryl groups substituted on the siloxane chain (R⁹³) or at the ends of the siloxane chains Z⁸ can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. Suitable Z⁸ groups include hydroxy, methyl, methoxy, ethoxy, propoxy, and aryloxy. The two R⁹³ groups on the silicon atom may represent the same group or different groups. Preferably, the two R⁹³ groups represent the same group. Suitable R⁹³ groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. The polyalkylsiloxanes that can be used include, for example, polydimethylsiloxanes. These silicone compounds are available, for example, from the General Electric Company in their Viscasil^{®} and TSF 451 series, and from Dow Corning in their Dow Corning SH200 series.

The above polyalkylsiloxanes are available, for example, as a mixture with silicone compounds having a lower viscosity. Such mixtures have a viscosity of preferably from 1,000mPa·s to 100,000mPa·s, more preferably from 5,000mPa·s to 50,000mPa·s. Such mixtures preferably comprise: (i) a first silicone having a viscosity of from 100,000mPa·s to 30,000,000mPa·s at 25°C, preferably from 100,000mPa·s to 20,000,000mPa·s; and (ii) a second silicone having a viscosity of from 5mPa·s to 10,000mPa·s at 25°C, preferably from 5mPa·s to 5,000mPa·s. Such mixtures useful herein include, for example, a blend of dimethicone having a viscosity of 18,000,000mPa·s and dimethicone having a viscosity of 200mPa·s available from GE Toshiba, and a blend of dimethicone having a viscosity of 18,000,000mPa·s and cyclopentasiloxane available from GE Toshiba.

The other silicone compounds useful herein also include a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. The "silicone gums" will typically have a mass molecular weight in excess of 200,000, generally between 200,000 and 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. The silicone gums are available, for example, as a mixture with silicone compounds having a lower viscosity. Such mixtures useful herein include, for example, Gum/Cyclomethicone blend available from Shin-Etsu.

The silicone compounds may further be incorporated in the present composition in the form of an emulsion, wherein the emulsion is made by mechanical mixing, or in the stage of synthesis through emulsion polymerization, with or without the aid of a surfactant selected from anionic surfactants, nonionic surfactants, cationic surfactants, and mixtures thereof.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from 0.001% to 10%, preferably up to 5% by weight of the composition.

A wide variety of other additional components can be formulated into the present compositions. These include: cationic conditioning polymers including, for example, cationic celluloses such as polyquaternium-10, and cationic guar gums; low melting point oils having a melting point of less than 25°C including, for example, unsaturated fatty alcohols such as oleyl alcohol and ester oils such as pentaerythritol ester oils; polyethylene glycols; other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and Phenoxyethanol; pH adjusting agents, such as citric acid, sodium citrate, succinic acid, phosphoric acid, sodium hydroxide, sodium carbonate; salts, in general, such as potassium acetate and sodium chloride; perfumes; sequestering agents, such as ethylenediamine tetra acetic acid and its salts; and ultraviolet and infrared screening and absorbing agents such as octyl salicylate, octyl methoxycinnamate, benzophenone-3 and benzophenone-4.

### PRODUCT FORMS

The conditioning compositions of the present invention can be in the form of rinse-off products or leave-on products, and can be formulated in a wide variety of product forms, including but not limited to creams, gels, emulsions, mousses and sprays.

The conditioning composition of the present invention is especially suitable for rinse-off hair conditioner. Such compositions are preferably used by following steps:
(i) after shampooing hair, applying to the hair an effective amount of the conditioning compositions for conditioning the hair; and
(ii) then rinsing the hair.

In one aspect, the present invention may relate to the use of a first sucrose polyester together with a second sucrose polyester in an aqueous hair care composition to provide a friction reduction between hair.

In one further aspect, the present invention may relate to the use of a first sucrose polyester together with a second sucrose polyester in an aqueous hair care composition to provide hair manageability benefit such as volume control, easy of combing, and/or hair alignment.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention. Where applicable, ingredients are identified by chemical or CTFA name, or otherwise defined below.

**[Compositions]**

| Components | Ex.1 | Ex.2 | Ex.3 | Ex.4 |
|---|---|---|---|---|
| First Sucrose Polyester * 1 | 0.3 | 0.45 | 0.5 | - |
| Second Sucrose Polyester *2 | 0.7 | 1.05 | 0.5 | - |
| *As blend* | *(1.0)* | *(1.5)* | *(1.0)* | - |
| Behenyl trimethyl ammonium methyl sulfate | 1.4 | 1.4 | 1.4 | 1.4 |
| Dicetyl dimethyl ammonium chloride | 0.50 | 0.5 | 0.5 | 0.5 |
| Cetyl alcohol | 0.9 | 0.9 | 0.9 | 0.9 |
| Stearyl alcohol | 2.3 | 2.3 | 2.3 | 2.3 |
| Perfume | 0.4 | 0.4 | 0.4 | 0.4 |
| Panthenol | 0.03 | 0.03 | 0.03 | 0.03 |
| Panthenyl ethyl ether | 0.03 | 0.03 | 0.03 | 0.03 |
| Benzyl Alcohol | 0.4 | 0.4 | 0.4 | 0.4 |
| Methylchloroisothiazolinone and Methylisothiazolinone | 0.03 | 0.03 | 0.03 | 0.03 |
| Deionized Water | q.s. to 100% | | | |

**[Compositions]**

| Components | Ex. 5 | Ex. 6 | Ex. 7 | Ex. 8 |
|---|---|---|---|---|
| First Sucrose Polyester * 1 | 0.3 | - | 1.0 | - |
| Second Sucrose Polyester *2 | 0.7 | - | - | 1.0 |
| *(As blend)* | *(1.0)* | - | - | - |
| Behenyl trimethyl ammonium methyl sulfate | 1.5 | 1.5 | 1.5 | 1.5 |
| Behenamidopropyldimethylamine | - | - | - | - |
| 1-glutamic acid | - | - | - | - |
| Behenyl trimethyl ammonium chloride | - | - | - | - |
| Dicetyl dimethyl ammonium chloride | 0.5 | 0.5 | 0.5 | 0.5 |
| Distearyl dimethyl ammonium chloride | - | - | - | - |
| Cetyl alcohol | 0.9 | 0.9 | 0.9 | 0.9 |
| Stearyl alcohol | 2.3 | 2.3 | 2.3 | 2.3 |
| Aminosilicone *2 | 0.3 | 0.3 | 0.3 | 0.3 |
| Dimethicone blend *3 | - | - | - | - |
| Dimethicone/Cyclomethicone *4 | - | - | - | - |
| Perfume | 0.4 | 0.4 | 0.4 | 0.4 |
| Panthenol | 0.03 | 0.03 | 0.03 | 0.03 |
| Panthenyl ethyl ether | 0.03 | 0.03 | 0.03 | 0.03 |
| Benzyl Alcohol | 0.4 | 0.4 | 0.4 | 0.4 |
| Methylchloroisothiazolinone and Methylisothiazolinone | 0.03 | 0.03 | 0.03 | 0.03 |
| Deionized Water | q.s. to 100% | | | |

**[Compositions]**

| Components | Ex. A | Ex. B (made by a different method) | Ex. C |
|---|---|---|---|
| First Sucrose Polyester * 1 | 0.3 | 0.3 | - |
| Second Sucrose Polyester *2 | 0.7 | 0.7 | - |
| (As blend) | (1.0) | (1.0) | - |
| Behenyl trimethyl ammonium methyl sulfate | 1.5 | 1.5 | 1.5 |
| Dicetyl dimethyl ammonium chloride | 0.5 | 0.5 | 0.5 |
| Cetyl alcohol | 0.9 | 0.9 | 0.9 |
| Stearyl alcohol | 2.3 | 2.3 | 2.3 |
| Aminosilicone *2 | - | - | 0.2 |
| Perfume | 0.4 | 0.4 | 0.4 |
| Panthenol | 0.03 | 0.03 | 0.03 |
| Panthenyl ethyl ether | 0.03 | 0.03 | 0.03 |
| Benzyl Alcohol | 0.4 | 0.4 | 0.4 |
| Methylchloroisothiazolinone and Methylisothiazolinone | 0.03 | 0.03 | 0.03 |
| Deionized Water | q.s. to 100% | | |

### Definitions of Components

* 1 First Sucrose Polyester: Available as Sefose 1618H from P&G Chemicals, having a melting point of about 60°C, IV of about 3.
*2 Second Sucrose Polyester: Available as Sefose 1618U from P&G Chemicals, having a melting point of about -5°C, IV of about 135.
*2 Aminosilicone: Terminal aminosilicone which is available from GE having a viscosity 10,000mPa·s, and having following formula (III):

   (R₁)ₐG₃₋ₐ-Si-(-OSiG₂)ₙ-(-OSiG_{b}(R₁)_{2-b})ₘ-O-SiG₃₋ₐ(R₁)ₐ (III)

   wherein G is methyl; a is an integer of 1; m is 0; n is a number from about 400 to about 600; R₁ is a monovalent radical conforming to the general formula CqH₂qL, wherein q is an integer of 3 and L is -NH₂
*3 Dimethicone blend: a blend of dimethicone having a viscosity of 18,000,000mPa·s and dimethicone having a viscosity of 200mPa·s available from GE Toshiba
*4 Dimethicone/Cyclomethicone: a blend dimethicone having a viscosity of 18,000,000mPa·s and cyclopentasiloxane available from GE Toshiba

### Method of Preparation

The conditioning compositions of "Ex. 1" through "Ex. 8", Ex. A" and "Ex. C" as shown above can be prepared by any conventional method well known in the art. They are suitably made as follows:
Cationic surfactants, high melting point fatty compounds are added to water with agitation, and heated to 80°C. If included, preservatives are added. The mixture is cooled down to 60°C. If two or more sucrose polyesters are added, they are mixed in a separate vessel at 70°C. If included, sucrose polyesters are added to the mixture at 60°C. Then the mixture is cooled down to 30°C. If included, other components such as silicones and perfumes are added to the mixture with agitation.

The conditioning composition of "Ex. B" as shown above can be prepared by any conventional method well known in the art. They are suitably made as follows:
Cationic surfactants, high melting point fatty compounds are added to water with agitation, and heated to 80°C. If included, preservatives are added. Then the mixture is cooled down to 30°C. If two or more sucrose polyesters are added, they are mixed in a separate vessel at 70°C. If included, sucrose polyesters are added to the mixture at 30°C. If included, other components such as silicones and perfumes are added to the mixture with agitation.

### Conditioning benefits

With respect to the above compositions, conditioning benefits are evaluated by the following methods. Results of the evaluation are also shown in below Tables 1-3.

### Friction reduction on dry hair

Dry conditioning performance is evaluated by hair friction force measured by an instrument named Instron Tester (Instron 5542, Instron, Inc,; Canton, Mass., USA). 2g of the composition is applied to 20g of hair sample. After spreading the composition on the hair sample, rinsing it with warm water for 30 seconds, and the hair sample is left to dry over night. The friction force (g) between the hair surface and a urethane pad along the hair is measured.
A1, A2 or A3: Above 10% (excluding 10%) to 20% reduction of Friction force, compared to Control (C1, C2 or C3)
B1, B2 or B3: Above 5% (excluding 5%) to 10% reduction of Friction force, compared to Control (C1, C2 or C3)
C1, C2 or C3: Control or Equal to Control
D1, D2 or D3: Above 5% (excluding 5%) to 10% increase of Friction force, compared to Control (C1, C2 or C3)
E1, E2 or E3: Above 10% (excluding 10%) to 20% increase of Friction force, compared to Control (C1, C2 or C3)
F1, F2 or F3: Above 20% (excluding 10%) to 30% increase of Friction force, compared to Control (C1, C2 or C3)

**Table 1**

| | Ex.1 | Ex.2 | Ex.3 | Ex. 4 |
|---|---|---|---|---|
| Friction reduction on dry hair | A1 | B 1 | C1 | C1 |

**Table 2**

| | Ex. 5 | Ex. 6 | Ex. 7 | Ex. 8 |
|---|---|---|---|---|
| Friction reduction on dry hair | - | C2 | C2 | C2 |

**Table 3**

| | Ex. A | Ex. B (made by a different method) | Ex. C |
|---|---|---|---|
| Friction reduction on dry hair | C3 | F3 | C3 |

The embodiments disclosed and represented by the previous "Ex. 1" through "Ex. 3", "Ex. 5" and "Ex. A" are hair conditioning compositions of the present invention which are particularly useful for rinse-off use. Such embodiments have many advantages. For example, they provide conditioning benefits, especially friction reduction on dry hair.

For example, comparison among Ex. 1, Ex. 2 and Ex. 3 shows that preferred ratio of the first and second sucrose polyesters and preferred level of the first and second sucrose polyesters provide better friction reduction.

For example, comparison between Ex. 5 and Ex. 7 and 8 shows that the use of both of the first and second sucrose polyesters provides improved friction reduction on dry hair, compared to the use of each of the first or second sucrose polyester.

For example, comparison between Ex. A and Ex. B shows that the preferred process provides improved friction reduction on dry hair, compared to another process.

## Claims

1. A hair care composition comprising:
a) at least one first sucrose polyester has a melting point greater than 50°C, and an iodine value (IV) of from 0.1 to 10;
b) at least one second sucrose polyester has a melting point lower than 10°C, and an iodine value (IV) of from above 100 to 150; and
c) an aqueous carrier;
wherein the weight ratio of the first sucrose polyester to the second sucrose polyester is from 1:1.6 to 1:3;
wherein the first sucrose polyester and the second sucrose polyester have Esterification Degree of, respectively, from 4 to 8; and
wherein the first sucrose polyester and the second sucrose polyester respectively have a fatty chain length of from 12 to 30 carbon atoms.

2. The hair care composition of Claim 1, wherein the total amount of the first and second sucrose polyester is from 0.1% to 10%, preferably from 0.6% to 2% by weight of the composition.

3. The hair care composition of any of the preceding claims, wherein the first sucrose polyester and the second sucrose polyester have Esterification Degree of, respectively, from 6 to 8.

4. The hair care composition of any of the preceding claims, wherein the first sucrose polyester and the second sucrose polyester respectively have a fatty chain length of from 16 to 22 carbon atoms.

5. The hair care composition of any of the preceding claims, wherein said composition further comprises a fatty alcohol having from 14 to 30 carbon atoms and a surfactant, preferably a cationic surfactant, more preferably a cationic surfactant comprising a mono-long alkyl cationic surfactant and a di-long alkyl cationic surfactant, and preferably wherein the fatty alcohol, the cationic surfactant, and the aqueous carrier form a gel matrix.

6. The method of preparing the composition of any of the preceding claims, wherein the first sucrose polyester is added to the carrier at a temperature which is higher than the melting point of the first sucrose polyester, and wherein the second sucrose polyester is added to the carrier at a temperature which is higher than the melting point of the second sucrose polyester.

7. The method of preparing the composition of any of claims 1 to 5, wherein the first and second sucrose polyesters are premixed to form a homogenous blend, and added to the carrier at a temperature which is higher than the melting point of the blend.

8. The method of preparing the composition of Claim 7, wherein said composition further comprises a fatty alcohol having from 14 to 30 carbon atoms and a cationic surfactant, wherein the fatty alcohol, the cationic surfactant, and the aqueous carrier form a gel matrix, and wherein the blend is added prior to the formation of gel matrix.

9. Use of an aqueous hair care composition comprising:
a first sucrose polyester, which has a melting point greater than 50° C and an iodine value (IV) of from 0.1 to 10, together with
a second sucrose polyester, which has a melting point of lower than 10°C and an iodine value (IV) of from 100 to 150,
in a weight ratio of the first sucrose polyester to the second sucrose polyester from 1: 1.6 to 1:3,
wherein the first sucrose polyester and the second sucrose polyester have Esterification Degree of, respectively, from 4 to 8; and
wherein the first sucrose polyester and the second sucrose polyester respectively have a fatty chain length of from 12 to 30 carbon atoms to provide a friction reduction between hair.

## Patentansprüche

1. Haarpflegezusammensetzung, umfassend:
a) mindestens einen ersten Saccharose-Polyester mit einem Schmelzpunkt von über 50 °C und einem Iod-Wert (IV) von 0,1 bis 10;
b) mindestens einen zweiten Saccharose-Polyester mit einem Schmelzpunkt von unter 10 °C und einem Iod-Wert (IV) von über 100 bis 150; und
c) einen wässrigen Träger;
wobei das Gewichtsverhältnis des ersten Saccharose-Polyesters zum zweiten Saccharose-Polyester von 1:1,6 bis 1:3 beträgt;
wobei der erste Saccharose-Polyester und der zweite Saccharose-Polyester einen Veresterungsgrad von jeweils 4 bis 8 aufweisen; und
wobei der erste Saccharose-Polyester und der zweite Saccharose-Polyester eine Fettkettenlänge von jeweils 12 bis 30 Kohlenstoffatomen aufweisen.

2. Haarpflegezusammensetzung nach Anspruch 1, wobei die Gesamtmenge des ersten und zweiten Saccharose-Polyesters von 0,1 Gew.-% bis 10 Gew.-%, vorzugsweise von 0,6 Gew.-% bis 2 Gew.-%, der Zusammensetzung beträgt.

3. Haarpflegezusammensetzung nach einem der vorstehenden Ansprüche, wobei der erste Saccharose-Polyester und der zweite Saccharose-Polyester einen Veresterungsgrad von jeweils 6 bis 8 aufweisen.

4. Haarpflegezusammensetzung nach einem der vorstehenden Ansprüche, wobei der erste Saccharose-Polyester und der zweite Saccharose-Polyester eine Fettkettenlänge von jeweils 16 bis 22 Kohlenstoffatomen aufweisen.

5. Haarpflegezusammensetzung nach einem der vorstehenden Ansprüche, wobei die Zusammensetzung ferner einen Fettalkohol mit 14 bis 30 Kohlenstoffatomen und ein Tensid umfasst, vorzugsweise ein kationenaktives Tensid, mehr bevorzugt ein kationenaktives Tensid umfassend ein kationenaktives Tensid mit einer langen Alkylkette und ein kationenaktives Tensid mit zwei langen Alkylketten, und wobei vorzugsweise der Fettalkohol, das kationenaktive Tensid und der wässrige Träger eine Gelmatrix bilden.

6. Verfahren zum Herstellen der Zusammensetzung nach einem der vorstehenden Ansprüche, wobei der erste Saccharose-Polyester zum Träger bei einer Temperatur hinzugefügt wird, welche höher ist als der Schmelzpunkt des ersten Saccharose-Polyesters und wobei der zweite Saccharose-Polyester zum Träger bei einer Temperatur hinzugefügt wird, welche höher ist als der Schmelzpunkt des zweiten Saccharose-Polyesters.

7. Verfahren zum Herstellen der Zusammensetzung nach einem der Ansprüche 1 bis 5, wobei der erste und der zweite Saccharose-Polyester vorgemischt werden, um eine homogene Mischung zu bilden, und dem Träger bei einer Temperatur hinzugefügt werden, welche höher ist als der Schmelzpunkt der Mischung.

8. Verfahren zum Herstellen der Zusammensetzung von Anspruch 7, wobei die Zusammensetzung ferner einen Fettalkohol mit 14 bis 30 Kohlenstoffatomen und ein kationenaktives Tensid umfasst, wobei der Fettalkohol, das kationenaktive Tensid und der wässrige Träger eine Gelmatrix bilden, und wobei die Mischung vor dem Bilden der Gelmatrix hinzugefügt wird.

9. Verwenden einer wässrigen Haarpflegezusammensetzung, Folgendes umfassend:
einen ersten Saccharose-Polyester, welcher einen Schmelzpunkt von über 50 °C und einen Iod-Wert (IV) von 0,1 bis 10 aufweist, zusammen mit einem zweiten Saccharose-Polyester, welcher einen Schmelzpunkt von unter 10 °C und einen Iod-Wert (IV) von 100 bis 150 aufweist,
in einem Gewichtsverhältnis des ersten Saccharose-Polyesters zum zweiten Saccharose-Polyester von 1: 1,6 bis 1:3,
wobei der erste Saccharose-Polyester und der zweite Saccharose-Polyester einen Veresterungsgrad von jeweils 4 bis 8 aufweisen; und
wobei der erste Saccharose-Polyester und der zweite Saccharose-Polyester eine Fettkettenlänge von jeweils 12 bis 30 Kohlenstoffatomen aufweisen, um eine Reibungsreduktion zwischen Haaren bereitzustellen.

## Revendications

1. Composition pour soins capillaires comprenant :
a) au moins un premier polyester de saccharose a un point de fusion supérieur à 50 °C et un indice d'iode (IV) allant de 0,1 à 10 ;
b) au moins un deuxième polyester de saccharose a un point de fusion inférieur à 10 °C et un indice d'iode (IV) allant de plus de 100 à 150 ; et
c) un véhicule aqueux ;
dans laquelle le rapport pondéral du premier polyester de saccharose sur le deuxième polyester de saccharose va de 1:1,6 à 1:3 ;
dans laquelle le premier polyester de saccharose et le deuxième polyester de saccharose ont un degré d'estérification de respectivement 4 à 8 ; et
dans laquelle le premier polyester de saccharose et le deuxième polyester de saccharose ont respectivement une longueur de chaîne grasse allant de 12 à 30 atomes de carbone.

2. Composition pour soins capillaires selon la revendication 1, dans laquelle la quantité totale des premier et deuxième polyesters de saccharose va de 0,1 % à 10 %, de préférence de 0,6 % à 2 % en poids de la composition.

3. Composition pour soins capillaires selon l'une quelconque des revendications précédentes, dans laquelle le premier polyester de saccharose et le deuxième polyester de saccharose ont un degré d'estérification de respectivement 6 à 8.

4. Composition pour soins capillaires selon l'une quelconque des revendications précédentes, dans laquelle le premier polyester de saccharose et le deuxième polyester de saccharose ont respectivement une longueur de chaîne grasse allant de 16 à 22 atomes de carbone.

5. Composition pour soins capillaires selon l'une quelconque des revendications précédentes, dans laquelle ladite composition comprend en outre un alcool gras possédant 14 à 30 atomes de carbone et un agent tensioactif, de préférence un agent tensioactif cationique, plus préférablement un agent tensioactif cationique comprenant un agent tensioactif cationique mono-alkyle long et un agent tensioactif cationique di-alkyle long, et de préférence dans laquelle l'alcool gras, l'agent tensioactif cationique et le véhicule aqueux forment une matrice de gel.

6. Procédé de préparation de la composition selon l'une quelconque des revendications précédentes, dans lequel le premier polyester de saccharose est ajouté au véhicule à une température qui est supérieure au point de fusion du premier polyester de saccharose, et dans lequel le deuxième polyester de saccharose est ajouté au véhicule à une température qui est supérieure au point de fusion du deuxième polyester de saccharose.

7. Procédé de préparation de la composition selon l'une quelconque des revendications 1 à 5, dans lequel les premier et deuxième polyesters de saccharose sont prémélangés pour former un mélange homogène, et ajoutés au véhicule à une température qui est supérieure au point de fusion du mélange.

8. Procédé de préparation de la composition selon la revendication 7, dans lequel ladite composition comprend en outre un alcool gras possédant 14 à 30 atomes de carbone et un agent tensioactif cationique, dans lequel l'alcool gras, l'agent tensioactif cationique et le véhicule aqueux forment une matrice de gel, et dans lequel le mélange est ajouté avant la formation de la matrice de gel.

9. Utilisation d'une composition pour soins capillaires aqueuse comprenant :
un premier polyester de saccharose, qui a un point de fusion supérieur à 50 °C et un indice d'iode (IV) allant de 0,1 à 10, conjointement avec
un deuxième polyester de saccharose, qui a un point de fusion inférieur à 10 °C et un indice d'iode (IV) allant de 100 à 150,
dans un rapport pondéral du premier polyester de saccharose sur le deuxième polyester de saccharose allant de 1:1,6 à 1:3,
dans laquelle le premier polyester de saccharose et le deuxième polyester de saccharose ont un degré d'estérification de respectivement 4 à 8 ; et
dans laquelle le premier polyester de saccharose et le deuxième polyester de saccharose ont respectivement une longueur de chaîne grasse allant de 12 à 30 atomes de carbone pour assurer une réduction des frottements entre les cheveux.
